# EUROPEAN PATENT SPECIFICATION

(11) **EP 0 378 671 B1**
(45) Date of publication and mention of the grant of the patent: **12.03.1997**
(21) Application number: 89908616.9
(22) Date of filing: 30.06.1989
(51) Int. Cl.: C07K 7/08, C07K 14/62, C07K 14/65

(54) **INSULINOMIMETIC AND INSULIN RECEPTOR BINDING SITE PEPTIDES**
INSULINOMIMETRISCHE EIGENSCHAFTEN UND INSULIN-REZEPTOR-BINDESTELLEN-PEPTIDE
PEPTIDES A SITE DE LIAISON DE RECEPTEURS D'INSULINE ET A PROPRIETES INSULINOMIMETIQUES

(30) Priority: 30.06.1988 US 213918; 30.12.1988 US 292099
(43) Date of publication of application: 25.07.1990
(73) Proprietor: CITY OF HOPE, Duarte California 91010-0269 (US)
(72) Inventor: DE MEYTS, Pierre, Pasadena, CA 91107 (US)
(74) Representative: Dowden, Marina
(86) International application number: US8902830
(87) International publication number: WO9000562

(56) References cited:
- JP-A-62 281 897
- US-A- 4 761 371
- THE EMBO JOURNAL, vol. 5, no. 10, 1986, Eynsham (GB); A. ULLRICH et al., pp. 2503-2512
- CELL, vol. 40, April 1985; Y. EBINA et al., pp. 747-758
- NATURE, vol. 313, 28 February 1985, A. ULLRICH; pp. 756-761
- CHEMICAL ABSTRACTS, vol. 99, no. 7, 1983, Columbus, OH (US); T. BLUNDELL, p. 64, no. 48026j
- CHEMICAL ABSTRACTS, vol. 105, no. 23, 1986, Columbus, OH (US); F. YAMAGUCHI, p. 126, no. 203833h

## Description

### RELATED APPLICATIONS

This application is a continuation-in-part of application Serial No. 213,918 filed June 30, 1988 entitled "Insulin Receptor Binding Site" and also a continuation-in-part of application Serial No. 292,099 filed December 30, 1988 entitled "Insulinomimetic and Insulin Receptor Binding Site Peptides". The specifications, figures and claims of these applications are incorporated into this application by reference.

### FIELD OF THE INVENTION

This invention relates to the amino acid residue sequences which bind the human insulin molecule or the insulin-like growth factor I (IGF-I) molecule and to linear peptides endowed with insulinomimetic properties which include the same or similar sequences.

### BACKGROUND OF THE INVENTION

The cDNA of both the insulin and the IGF-I receptors have been cloned and sequenced. Expression in mammalian cells of biologically active receptors has been achieved. See United States patent 4,761,371; Ebina, et al., Cell 46:747-758 (1985); Ullrich, et al., Nature 313:756-761 (1985); and Ullrich, et al., EMBO J. 5:2503-2512 (1986). Ebina and Ullrich utilize different sequence numbers. The Ullrich sequence numbers are used exclusively herein.

The receptors are glycoproteins which consist of a heterotetramer of two extracellular α subunits and two transmembrane β subunits which have both an extracellular and an intracellular portion. The α subunits contain the insulin binding site. It is plausible that each α subunit may contain one binding site. The insulin receptor has homology with the IGF-I receptor and with the epidermal growth factor receptor, the human c-erb-2 oncogene, and the v-ros oncogene-encoded tyrosine kinase.

Knowledge of receptor structures is limited to the primary sequence deduced from cDNA cloning. This information has produced few clues as to the precise localization of the insulin or IGF-I binding domain of the α subunits, each of which contains more than 700 amino acid residues. Nor is definitive information concerning the secondary or tertiary structure of the receptors available.

The discovery of insulin-like peptides has long been a major challenge to the diabetes related pharmaceutical industry. Apparently, the only previous description of a linear peptide purportedly having insulin-like activity is found in publications by Weitzel, et al., Hoppe-Seyler's Z. Physiol. Chem. 352:1005-1013 (1971); ibid., 1735-1738 (1971); ibid., 357:187-200 (1976). These publications report that the terminal portion of insulin's B-chain is active. This claim was never confirmed by others. Per contra, the C-terminal portion of the B-chain (B23-30) has been used as a negative control in experiments which reveal the insulin-like peptides of this invention.

### SUMMARY OF THE INVENTION

This invention entails identification of receptor domains involved in the binding of insulin or IGF-I molecules, the amino acid residue sequences of such domains, and their secondary and tertiary structure. It includes natural and synthetic fragments of such domains and sequences which are effective in implementation of the binding and recognition of the insulin or IGF-I molecule by the receptors, as well as physical and graphic representations of these domains.

A seminal aspect of the invention is the discovery that the human insulin receptor domain is insulinomimetic per se and that it includes shorter sequences which are similarly endowed. Synthetic and purified natural amino acid residue sequences, many of which are insulinomimetic and constitute, in whole or in part, a binding site for the human insulin and IGF-I molecules, are provided.

The invention includes the discovery that the receptor domain strongly aggregates in solution with apparent capability to bind an intact insulin receptor on cells and activate it with resulting insulin-like activity, e.g., the activation of lipogenesis in isolated rat adipocytes.

Another aspect of the invention includes derivatives and modifications of such peptides, for example, shortened sequences of minimal structure required for binding or insulinomimetic action and cyclization or derivatization to impart or enhance solubility in the gastrointestinal tract.

The insulinomimetic peptides of the invention and derivatives and modifications thereof may be administered to diabetic patients in lieu of insulin, either subcutaneously, intranasally or orally. These peptides of the invention are useful in biochemical experiments in vitro and in vivo to study the mechanisms of insulin activity.

Exemplified hereinafter, are physical and graphical representations of the spatial or three-dimensional structure of these peptides. A possible use of such representations is as templates for the design of insulinomimetic and other drugs.

### DESCRIPTION OF THE FIGURES

Figure 1A is a computer-generated graphic model of the insulin dimer interface.

Figure 1B is a computer-generated graphic model of the insulin sequence B19-30 of one insulin monomer in the dimer interface replaced by sequence 83-94 of the insulin receptor.

Figure 2 depicts the homology of various receptor fragments with the C-terminal end of the insulin B-chain.

Figure 3 is a computer-generated secondary structure and hydropathy profile of the α subunit.

Figures 4A-4D are computer-generated graphic models from four different angles of the α subunit sequence which includes residues 83-94.

Figures 5A-5B are similarly computer-generated graphic models from two different angles of the entire domain including residues 83-94 in a β-sheet structure and residues 95-103 in an α-helix consistent with Figure 3.

Figure 6 is an insulin competition curve with synthetic receptor peptide.

Figure 7 depicts homology between C-terminal ends of insulin and IGF-I B-chain and receptors for insulin, IGF-I and EGF.

Figures 8A and 8B are graphic representations of the insulin-insulin dimer pair and of the insulin-insulin receptor domain including the residues 83-94. Figure 8C is a graphic representation of the IGF-I receptor domain-insulin pair. In each of the figures, the graphic representation of "insulin" appears on the right.

Figures 9A-D are graphic models from four different angles of the IGF-I receptor domain, including the residues 77-97.

Figure 10 depicts the insulinomimetic effect of certain receptor peptides.

Figure 11 depicts the range of concentration over which a peptide of Sequence I binds to insulin.

Figure 12 shows that the lipogenetic activity of a Sequence V peptide is inactivated by the polyclonal anti-insulin antibody Sigma #1-8510.

Figure 13 shows that the stimulation of fat cell lipogenesis by the peptide of Sequence V is blocked by staurosporine and sphingosine.

Biograph computer software was utilized to generate all computer-generated graphic models included in the Figures.

### Identification of the Insulin Receptor Binding Domain

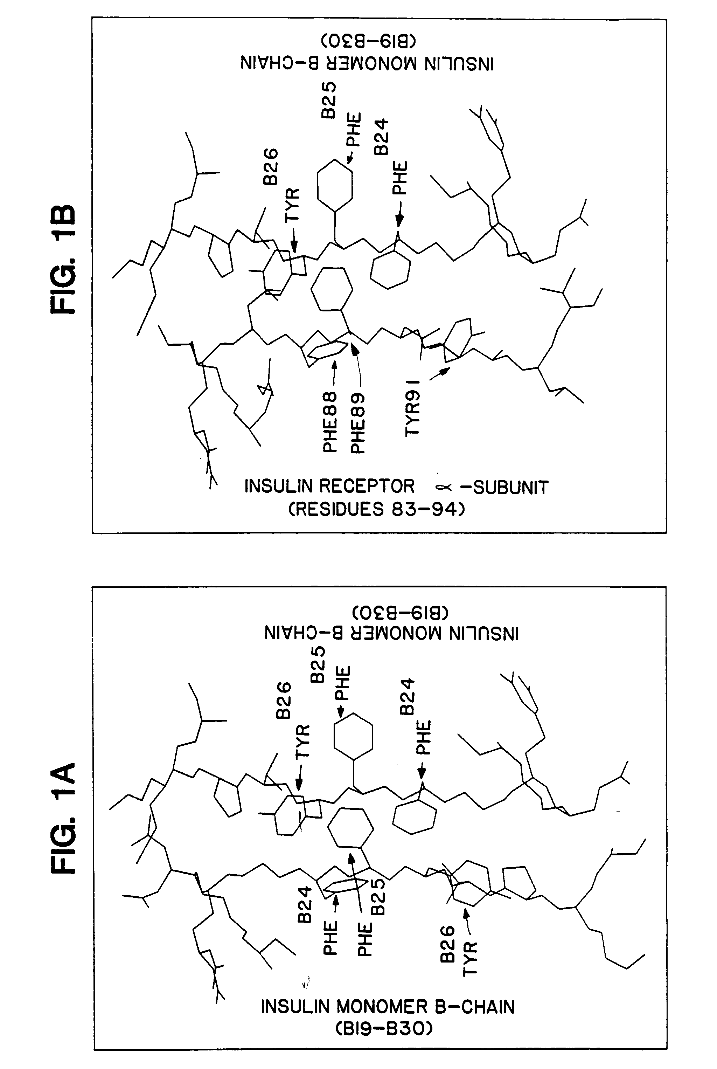

The insulin molecule B-chain residues B23-26 Gly-Phe-Phe-Tyr are involved directly in a receptor binding domain. It is also known that the C-terminal portions of the monomeric insulin B-chains bind inter se to form the insulin dimer. From a study of the dimer interface (see Figure 1A), it may be deduced that a receptor sequence for interaction in a similar fashion with the same C-terminal portion of the insulin monomer
(i) would contain two or three Phe or Tyr chains;
(ii) may have some homology with the C-terminal end of the insulin B-chain;
(iii) would be in a hydrophobic environment; and
(iv) would be in a β-sheet secondary structure.

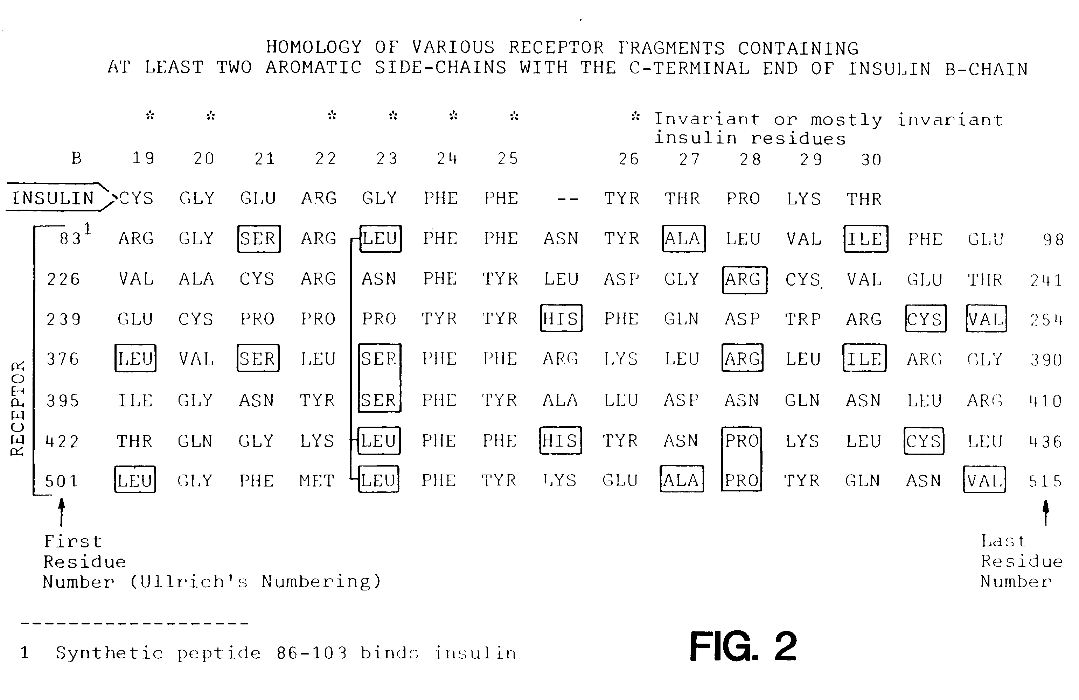

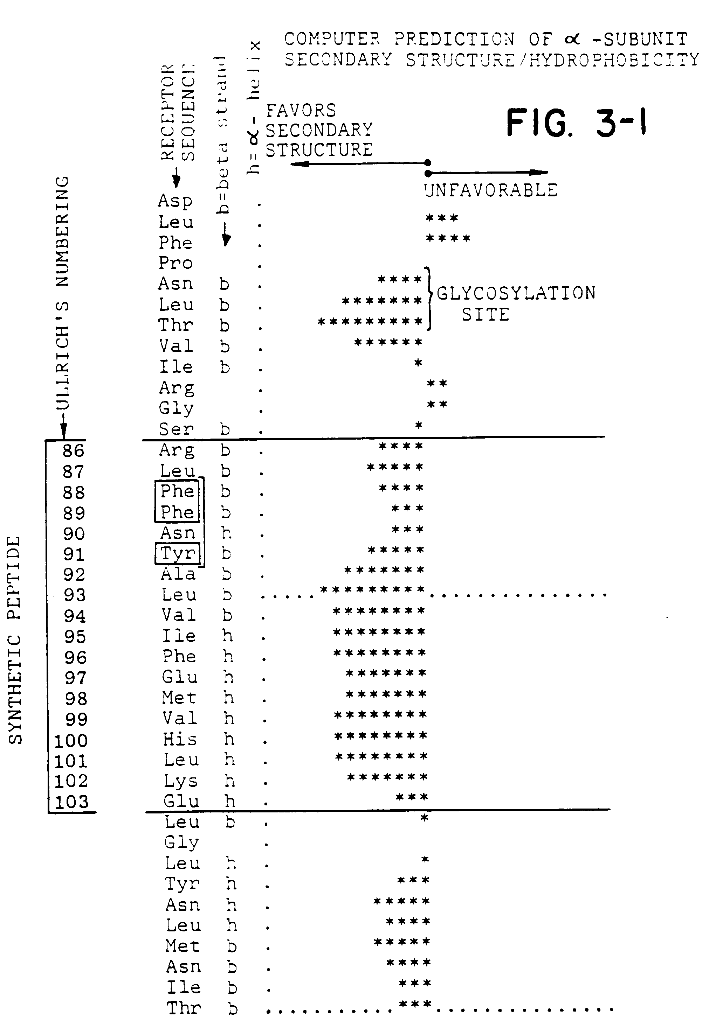

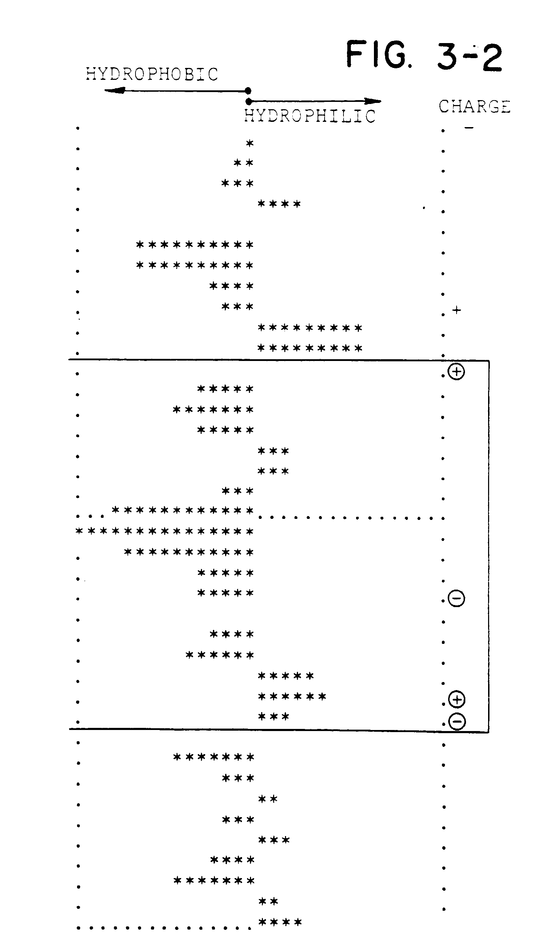

Inspection of the known sequence of the receptor α subunit reveals various stretches containing at least two or three Phe or Tyr chains in close proximity. Of these, the 88-91 (Phe-Phe-Asn-Tyr) stretch was selected because:
(i) As Figure 2 shows, the sequence 84 to 91 has 5 residues homologous to the invariant or mostly invariant insulin residues. 20-26.
(ii) As Figure 3 shows, a computer-generated secondary structure and hydropathy profile of the α subunit indicates that the receptor segment 78-94 containing the Phe-Phe-Asn-Tyr sequence is a β-sheet followed by a helical portion 95 to 103 and that the whole sequence is largely hydrophobic.

A synthetic 18 amino acid peptide corresponding to the receptor sequence 86-103 was synthesized on a solid support utilizing the p-alkoxybenzyl ester anchoring linkage of Wang, S.S., J.Am.Chem.Soc. 95:1328-1333 (1973). The amino acids were amino-protected by the N-fluorneylmethoxycarbonylamino (Fmoc) group. The side chains were protected with t-butyl or other appropriate protecting groups. After synthesis the peptide was cleaved from the solid support by trifluoroacetic acid containing suitable solvents and other reagents to protect the peptide during this cleavage. The peptide was then passed through a Sephadex-G-10 column and final purification was achieved by using a C-18 column on an HPLC system. The exact molecular weight of the peptide was verified on a high resolution mass spectrometer. It was found to be highly hydrophobic as evidenced by substantial insolubility in less than 90% dimethylsulfoxide (DMSO). In water at high concentrations, the peptide formed a gel-like suspension and eventually precipitated as transparent crystalline-appearing structures.

The amino acid residues in this peptide are in the sequence depicted by the following Sequence I:

ARG-LEU-PHE-PHE-ASN-TYR-ALA-LEU-VAL-ILE-PHE-GLU-MET-
 86  87  88  89  90  91  92  93  94  95  96  97  98

VAL-HIS-LEU-LYS-GLU
 99 100 101 102 103

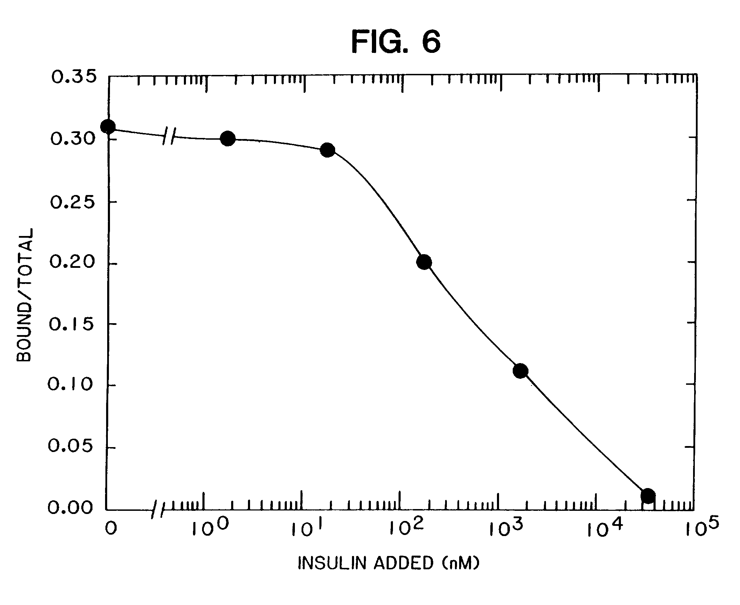

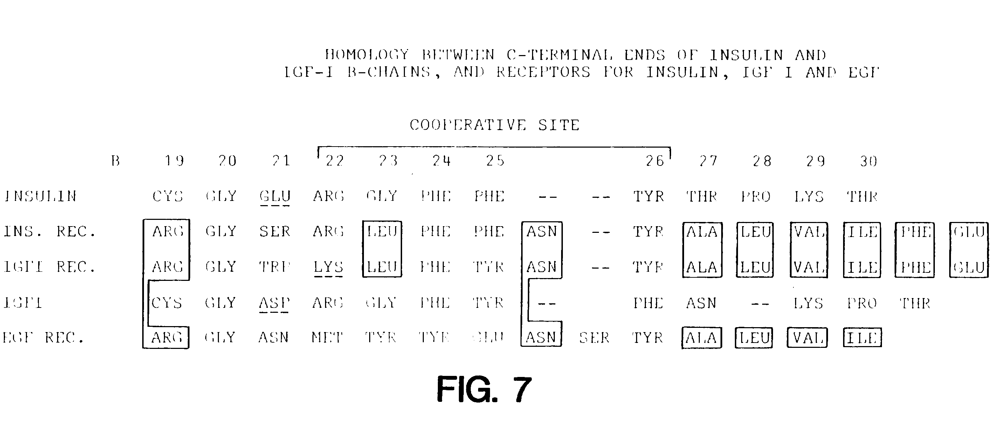

To test for insulin binding, a water suspension of the peptide was incubated at room temperature with a tracer of ¹²⁵I-insulin followed by simple centrifugation. The peptide was found to bind up to 30% of the tracer. The binding was displacable by an excess of unlabelled insulin. See Figure 6. The apparent dissociation constant was ∼6 x 10⁻⁷M. Non-specific binding was negligible. The peptide also bound ¹²⁵I-IGF I, but less well, while ¹²⁵I-EGF (epidermal growth factor) and ¹²⁵I-hGH (human growth hormone) showed no specific binding. These data correspond well to the predictions from sequence homologies in Figure 7.

This invention includes modifications of Sequence I in which additional residues corresponding to preceding and succeeding portions of the receptor α subunit are present. For example, the invention includes a Sequence II which also includes residues 83 to 85 of the insulin receptor α subunit as follows:

ARG-GLY-SER-ARG-LEU-PHE-PHE-ASN-TYR-ALA-LEU-VAL-ILE-
 83  84  85  86  87  88  89  90  91  92  93  94  95

PHE-GLU-MET-VAL-HIS-LEU-LYS-GLU
 96  97  98  99 100 101 102 103

The invention also includes modification of Sequence I in which one or more additional residues are added at one or both ends of the sequence to increase solubility. Specifically, the invention contemplates a Sequence III which includes 
(LYS)x-ARG-LEU-PHE-PHE-ASN-TYR-ALA-LEU-VAL-ILE-PHE-GLU-
86 87 88 89 90 91 92 93 94 95 96 97

MET-VAL-HIS-LEU-LYS-GLU-(LYS)y
98 99 100 101 102 103
 in which x and y are each 0, 1 or 2 with the provision that at least x or y is 1.

An additional Sequence IV has been synthesized for like solubility reasons:

LYS-ARG-GLY-SER-ARG-LEU-PHE-PHE-ASN-TYR-ALA-LEU-
83 84 85 86 87 88 89 90 91 92 93

VAL-ILE-PHE-GLU-MET-VAL-HIS-LEU-LYS-LYS
94 95 96 97 98 99 100 101 102

As appears from inspection, Sequence IV was produced by including residues 83-85, adding one LYS at the N-terminal, and replacing the negatively charged GLU at 103 by LYS, thus producing two positively-charged residues, LYS-ARG and LYS-LYS, at the N and C terminals, respectively.

Sequences V, VI and VII are modifications of Sequence IV achieved by replacing PHE-PHE at 88-89 with LEU-PHE (Sequence V), PHE-LEU (Sequence VI) and LEU-LEU (Sequence VII).

The invention further includes Sequences I to VII coupled to beads of polystyrene or other solid supports for use in solid phase assays and to keyhole limpet hemocyanin for use in the production of sequence antibodies. Included as well is the synthesis of an oligonucleotide corresponding to the Sequence I or II peptides and their expression in E. coli as part of a fusion protein with, for example, β-galactosidase or dihydrofolate reductase. Fusion may be to a synthetic IgG-binding domain from staphylococcus aureus protein A (see Lowenalder, B., et al. Gene 58:87-97 (1987)) to generate sequence antibodies for use in the performance of physical studies such as x-ray crystallography.

Figure 1B illustrates replacement of the side chains of residues CYS^{B19}, GLU^{B21}, GLY^{B23} and B26-30 of one of the insulin monomers shown in Figure 1A by the side chains of the residues occupying homologous positions in the insulin receptor sequence--specifically by ARG 83, SER 85, LEU 87 and ASN-TYR-ALA-LEU-VAL. More specifically, Figure 1B shows a molecular graphic model of the insulin sequence B19-B30 of one insulin monomer in the dimer interface replaced by sequence 83-94 of the insulin receptor. The similarity to the insulin dimer interface, Figure 1A, is striking.

This data indicates that residues 83 to 94 of the insulin receptor a subunit includes at least an effective portion of a domain involved in binding the active site of the insulin molecule.

### Identification of the IGF-I Receptor Binding Domain

The involvement of Sequence VII of the IGF-I receptor residues 77-97 homologous to insulin receptor sequence 83-103 in IGF-I binding is evidenced by the following:
(i) Insulin and IGF-I bind to each other's receptors.
(ii) Sequence B23-26 of the insulin molecule is conserved in IGF-I molecule (GLY-PHE-TYR-PHE instead of GLY-PHE-PHE-TYR).
(iii) Sequence 83-97 of insulin receptor is highly conserved in IGF-I receptor.
(iv) Synthetic peptide (Sequence I) binds IGF-I.

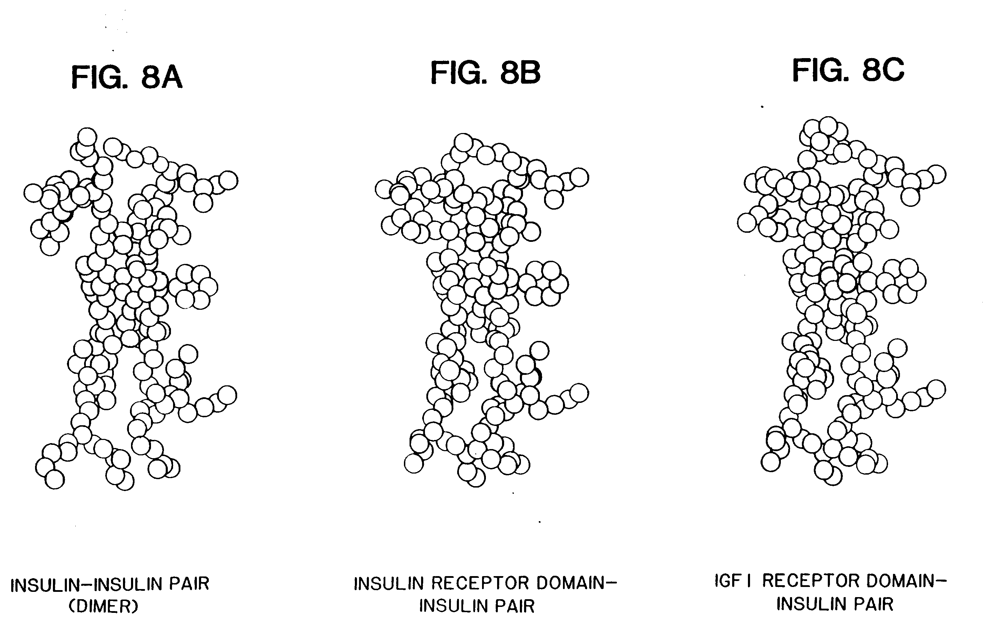

(v) Figure 8C, a graphic representation, illustrates replacement of the side chains of residues CYS^{B19}, GLU^{B21}, GLY^{B23} and B²⁶⁻³⁰ of one of the insulin monomers shown in Figure 1A by the side chains of the residues occupying homologous positions in the IGF-I receptor sequence--specifically by ARG, TRP, LYS, LEU and ASN-TYR-ALA-LEU-VAL, and also shows striking similarity to the insulin dimer interface. Accordingly, the invention also includes the following Sequence VIII of the IGF-I receptor:

ARG-GLY-TRP-LYS-LEU-PHE-TYR-ASN-TYR-ALA-LEU-VAL-ILE-
 77  78  79  80  81  82  83  84  85  86  87  88  89

PHE-GLU-MET-THR-ASN-LEU-LYS-ASP
 90  91  92  93  94  95  96  97

The invention may be utilised to provide physical and graphic representations of Sequence VIII to be used in the design of drugs.

### Demonstration of Insulinomimetic Properties

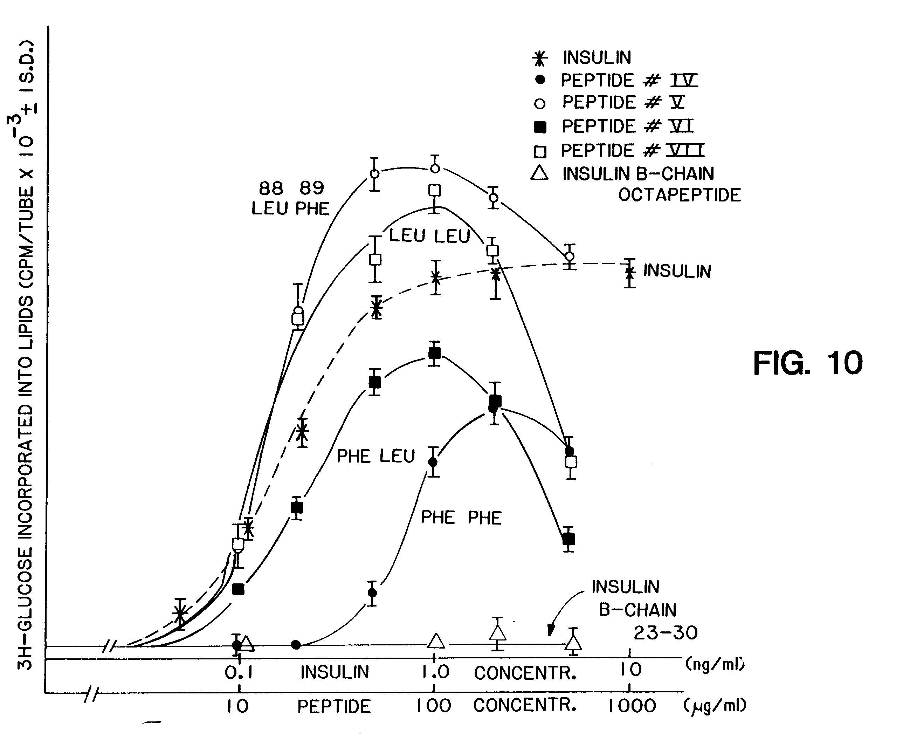

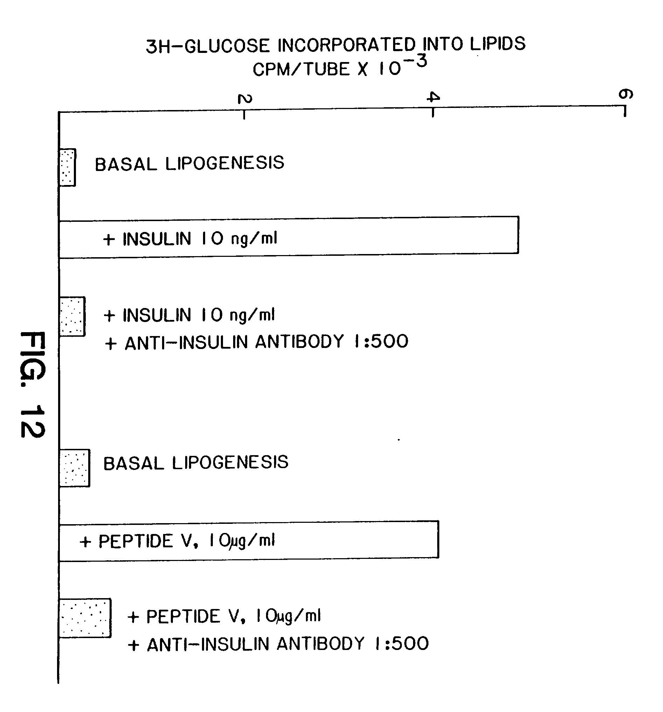

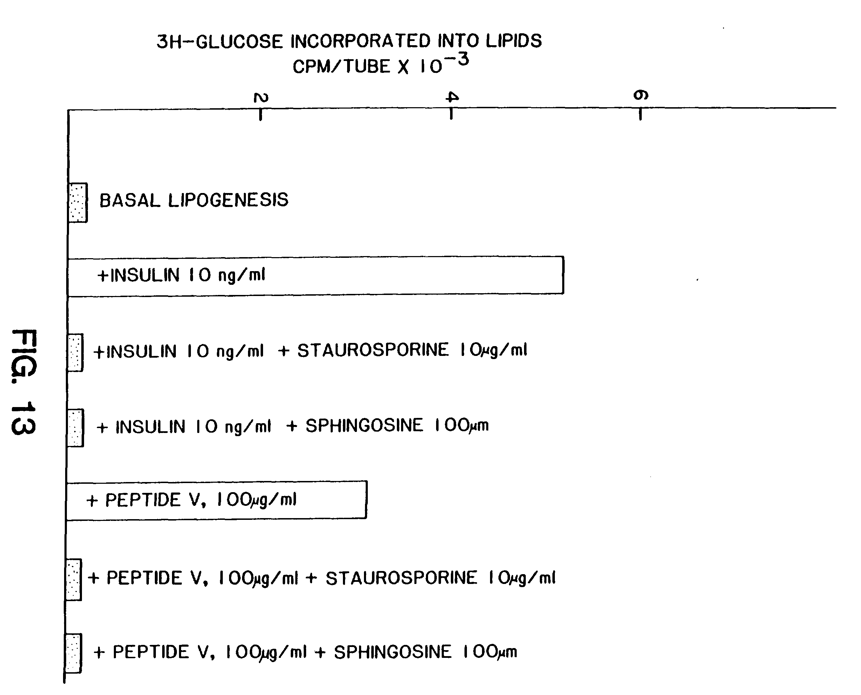

Insulinomimetic properties of the peptides of this invention are demonstrated by:
1. The ability, as compared to insulin, of these peptides to stimulate the incorporation of 3-[³H] glucose into the lipids of isolated rat adipocytes.
2. A demonstration that insulin octapeptide B23-30 is totally inactive in the same lipogenesis assay (Figure 10).
3. A demonstration that the lipogenetic activity of the peptides is inactivated by polyclonal insulin antibody suggesting structurally similar peptide and insulin epitopes (Figure 12).
4. The lipogenetic activity of the peptides, like that of insulin, is inhibited by kinase C inhibitors sphingosine and staurosporine suggesting similar pathways of action (Figure 13).
5. A peptide with insulin receptor sequence 81-91 is totally inactive suggesting that the region 92-103 is important for observation of the insulinomimetic effect. Further, an antibody against peptide sequence 81-91 does not block the lipogenetic effect of Sequence V.

### Example I

### Comparative Lipogenesis Assay

The ability of Sequences IV through VII to stimulate the incorporation of 3-[³H] glucose into lipids of isolated rat adipocytes was compared to insulin's.

The lipogenesis assay was conducted according to Smal, et al., J.Biol.Chem. 262:11071-11079 (1987). Dissected epididymal and retroperitoneal fat pads were digested under vigorous shaking at 37°C for 30 min with collagenase (1.0 mg/ml) in Krebs-Ringer-Heps (KRH) buffer, pH 7.4, 35 mg/ml dialyzed bovine serum albumin (BSA), 0.27 mM glucose. After filtration on cheesecloth and 4 washes in KRH with 10 mg/ml BSA, the adipocytes were preincubated for 4 hours at 37°C in the same buffer.

The lipogenesis assay was performed in triplicate in 6 ml polyethylene vials by adding successively 400 µl of adipocyte suspension (80 X l0³ cells/ml), 50 µl of KRH buffer pH 7.4 (1% BSA, 0.27 mM glucose) without hormone (basal lipogenesis) or with insulin or receptor-derived peptides at the indicated concentrations, and 50 µl D-[3-³H]-glucose in a total volume of 0.5 ml. The vials were incubated 2 hours at 37°C under gentle shaking. The incubation was interrupted by adding 5 ml/tube of toluene scintillator (1 liter toluene + 0.3 g of 1,4bis[2-(4-methyl-5 phenyloxazolyl)] benzene and 5 g of 2,5-diphenyloxazole under vigorous shaking (30s to break the cells) followed by a rest of at least 1 hour to allow extraction of lipids into the toluene phase before counting. The counting efficiencies for the different samples were measured by internal standardization with quenched tritiated standards. The incorporation of D-[3-³H]-glucose into lipids is expressed in cpm X 10⁻³/tube ± 1 standard deviation.

As shown in Figure 10, all four peptides stimulated lipogenesis to an extent close to insulin's effect; the relative potency of the four peptides varied to some extent from experiment to experiment perhaps due to a somewhat limited solubility. The concentration of peptides required was much higher than that of insulin (note the two different horizontal scales).

### Example II

### Peptide Binding Concentration Range

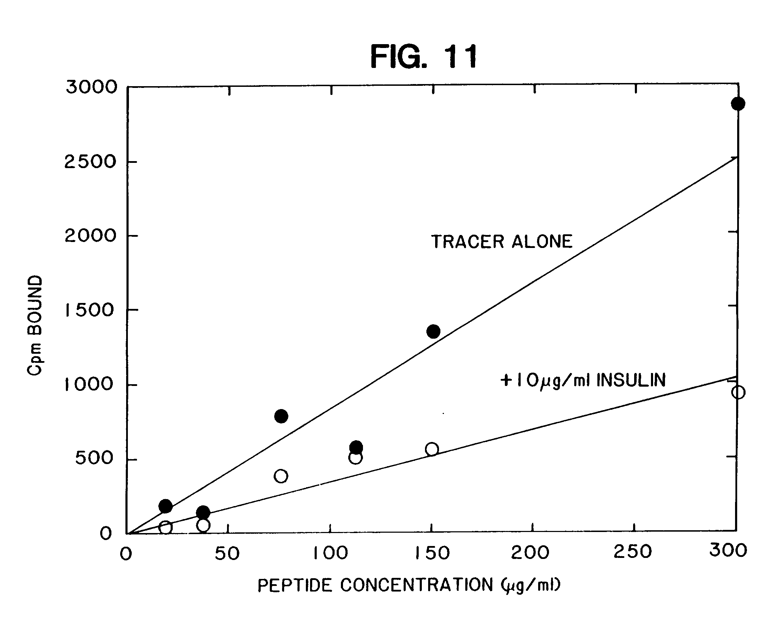

The range of concentration over which a peptide of Sequence I binds to insulin is shown by Figure 11 to be similar to the range of concentration over which peptides IV through VII are shown to be active by Figure 10.

To provide the data illustrated by Figure 11, ¹²⁵I-insulin (1 X 10⁻¹¹ M) was incubated overnight at room temperature in the absence or presence of 10 µg/ml unlabeled insulin with the indicated concentrations of peptide in assay buffer (100 mM Hepes, 120 mM NaCl, 5 mM KCL, 1.2 mM MgSO₄, 1 mM EDTA, 10 mM glucose, 15 mM NaC₂H₃O₂, 1% bovine serum albumin, pH 7.6). Bound and free tracer were separated by centrifuging the peptide suspension at 10,000 rpm for 10 min in a Beckman microfuge. The pellets were counted in a γ-counter.

### Example III

### Inhibition of Peptide Lipogenesis Activity by Anti-Insulin Antibody

Figure 12 shows that the lipogenetic activity of a Sequence V peptide is inactivated by the polyclonal anti-insulin antibody Sigma #1-8510.

To provide the data illustrated by Figure 12, a lipogenesis assay in the absence of hormone (basal), or with 10 µg/ml of insulin or 10 µg/ml of peptide V, in the absence or presence of anti-insulin antibody at a dilution of 1:500, was performed as described in detail with respect to Example I.

### Example IV

### Inhibition of Peptide Lipogenetic Activity by Kinase C Inhibitors

Co-pending application Serial No. 216,379 filed July 8, 1988 is entirely incorporated herein by reference. That application teaches that the stimulation of fat cell lipogenesis by insulin is blocked reversibly by the action of a kinase C inhibitor such as kinase C or staurosporine.

Figure 13 illustrates a like result with the peptide of Sequence V.

To generate the data depicted by Figure 13, a lipogenesis assay in the absence of hormone (basal), or with 10 µg/ml of insulin or 100 µg/ml of peptide V, in the absence or presence of kinase inhibitors staurosporine (10 µg/ml) or sphingosine (100 µM), was performed as described in detail with respect to Example I.

### Example V

### Activity of Insulin Receptor Sequence 92-103

This example indicates insulinomimetic activity of the insulin receptor sequence 92-103 (Sequence IX):

ALA-LEU-VAL-ILE-PHE-GLU-MET-VAL-HIS-LEU-LYS-GLU
92 93 94 95 96 97 98 99 100 101 102 103

The first aspect of this example is that the insulin receptor sequence 81 to 91 is totally inactive lipogenetically. Specifically, when peptide with sequence 81 to 91 is used instead of peptides IV through VII in the experiment described in Example I, lipogenesis is not stimulated above basal.

A second aspect of the example is that an antibody against peptide 81-91 is unable to block the effect of the peptide of Sequence V.

A further specific embodiment of this invention includes synthetic and purified peptide sequences corresponding to Sequence IX, and insulinomimetic drugs containing such peptides

The invention can be used to provide

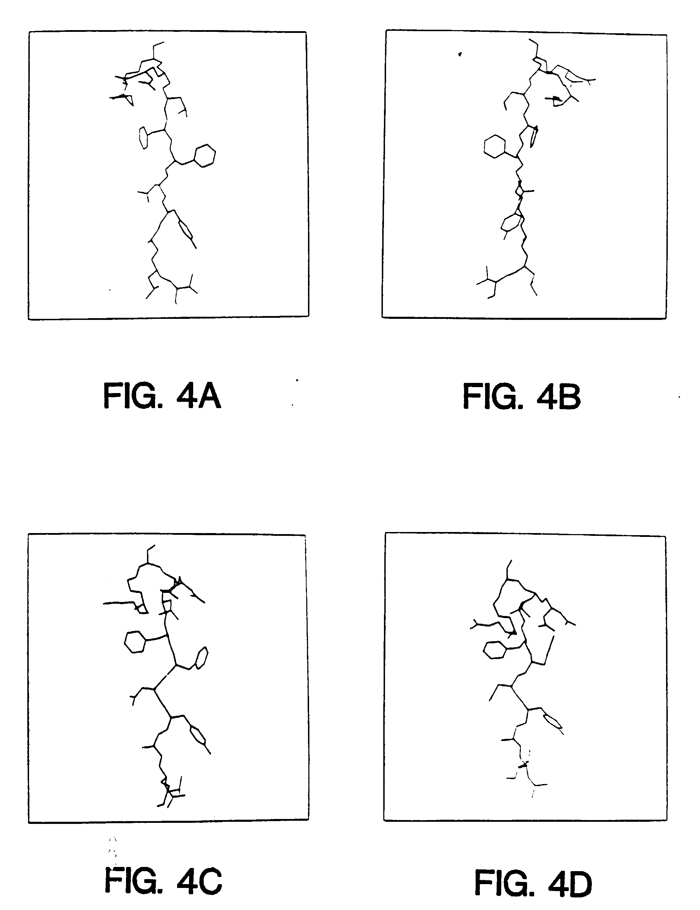

A three dimensional molecular model including residues 83-94 of the insulin receptor α subunit as depicted by any of Figures 4A, 4B, 4C or 4D;

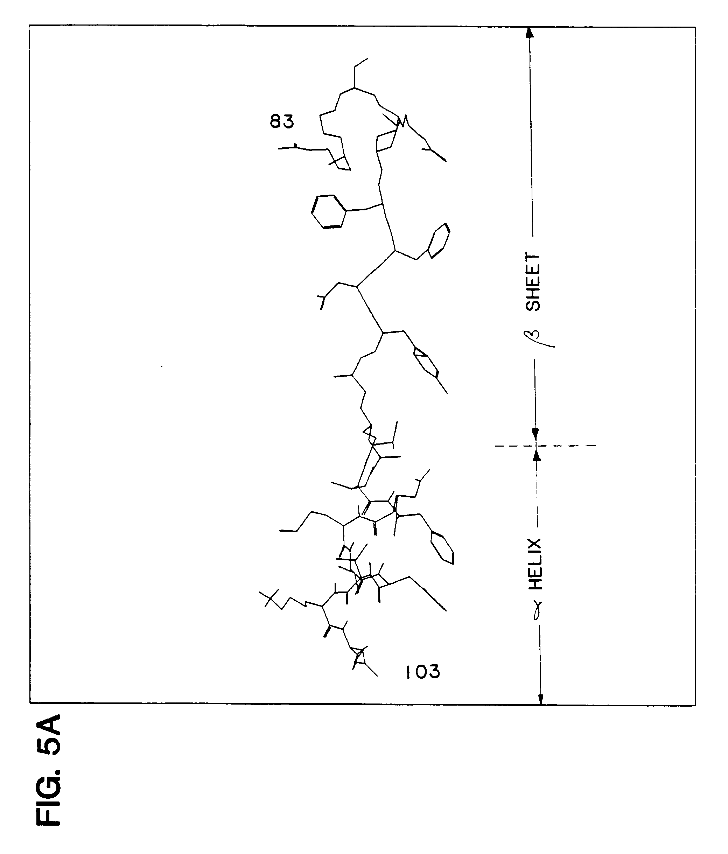

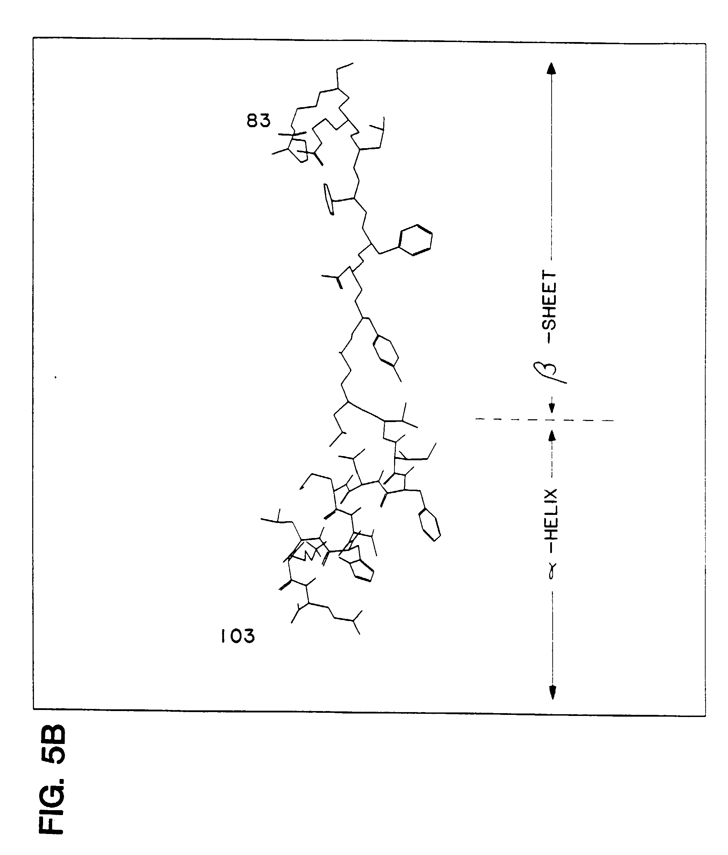

A three dimensional molecular model comprising at least so much of the graphic model depicted by Figures 4A, 4B, 4C, 4D, 5A or 5B as is effective to bind the human insulin molecule;

A graphical representation, which may be computer generated, of at least so much of residues 83-94 of the insulin receptor α subunit as is effective to bind the human insulin molecule;

The computer generated graphic representations depicted by Figures 4A, 4B, 4C or 4D;

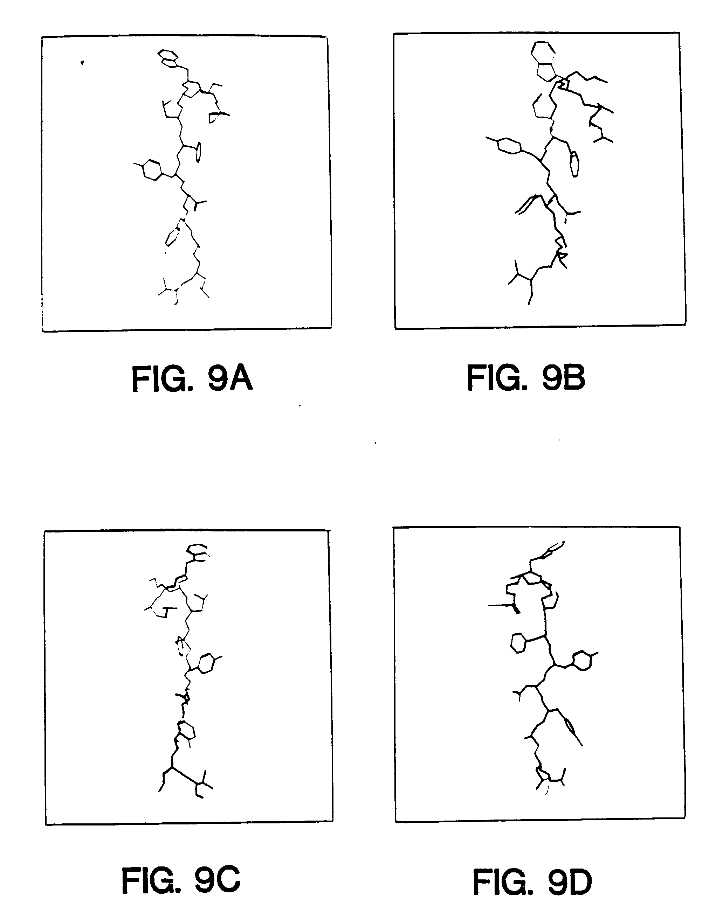

A three dimensional molecular model including residues 77-97 of the IGF-I receptor as depicted by any of Figures 9A, 9B, 9C or 9D;

A three dimensional molecular model comprising at least so much of the graphic model depicted by Figures 9A, 9B, 9C or 9D as is effective to bind the IGF-I molecule;

A graphic representation, which may be computer generated, of at least so much of residues 77-97 of the IGF-I receptor as is effective to bind the human insulin molecule;

The computer generated graphic representation depicted by Figures 9A, 9B, 9C and 9D, and A three-dimensional molecular model comprising at least so much of the graphic model depicted by Figure 5A or 5B as is effective to bind the human insulin molecule.

## Claims

1. A purified natural or a synthetic peptide whose amino acid sequence is Sequence I: 
Arg-Leu-Phe-Phe-Asn-Tyr-Ala-Leu-Val-Ile-Phe-Glu-Met-
86 87 88 89 90 91 92 93 94 95 96 97 98

Val-His-Leu-Lys-Glu
99 100 101 102 103
 or Sequence II: 
Arg-Gly-Ser-Arg-Leu-Phe-Phe-Asn-Tyr-Ala-Leu-Val-Ile-
 83  84  85  86  87  88  89  90  91  92  93  94  95

Phe-Glu-Met-Val-His-Leu-Lys-Glu
 96  97  98  99 100 101 102 103
 or Sequence III: 
(Lys)x-Arg-Leu-Phe-Phe-Asn-Tyr-Ala-Leu-Val-Ile-Phe-Glu-
86 87 88 89 90 91 92 93 94 95 96 97

Met-Val-His-Leu-Lys-Glu-(Lys)y
98 99 100 101 102 103
 in which x and y are each 0, 1 or 2 with the provision that at least x or y is 1
which binds the human insulin molecule and which has insulinomimetic activity, or an active fragment of any of Sequences I, II or III.

2. A non-naturally occurring peptide whose amino acid residue sequence is 
ARG-GLY-SER-ARG-LEU-PHE-PHE-ASN-TYR-ALA-LEU-VAL-ILE-
83 84 85 86 87 88 89 90 91 92 93 94 95
PHE-GLU-MET-VAL-HIS-LEU-LYS-GLU
96 97 98 99 100 101 102 103
 or 
(LYS)x-ARG-LEU-PHE-PHE-ASN-TYR-ALA-LEU-VAL-ILE-PHE-
86 87 88 89 90 91 92 93 94 95 96

GLU-MET-VAL-HIS-LEU-LYS-GLU-(LYS)y
97 98 99 100 101 102 103
 in which each of x and y is 0, 1 or 2, provided that at least one of x and y is 1
or 
LYS-ARG-GLY-SER-ARG-LEU-PHE-PHE-ASN-TYR-ALA-LEU-VAL-
83 84 85 86 87 88 89 90 91 92 93 94

ILE-PHE-GLU-MET-VAL-HIS-LEU-LYS-LYS
95 96 97 98 99 100 101 102
 or 
LYS-ARG-GLY-SER-ARG-LEU-LEU-PHE-ASN-TYR-ALA-LEU-VAL-
83 84 85 86 87 88 89 90 91 92 93 94
ILE-PHE-GLU-MET-VAL-HIS-LEU-LYS-LYS
95 96 97 98 99 100 101 102
 or 
LYS-ARG-GLY-SER-ARG-LEU-PHE-LEU-ASN-TYR-ALA-LEU-VAL-
83 84 85 86 87 88 89 90 91 92 93 94
ILE-PHE-GLU-MET-VAL-HIS-LEU-LYS-LYS
95 96 97 98 99 100 101 102
 or

LYS-ARG-GLY-SER-ARG-LEU-LEU-LEU-ASN-TYR-ALA-LEU-VAL-
83 84 85 86 87 88 89 90 91 92 93 94

ILE-PHE-GLU-MET-VAL-HIS-LEU-LYS-LYS
95 96 97 98 99 100 101 102

3. A purified or a synthetic fragment of the human insulin receptor α subunit which comprises Sequence I according to claim 1, said fragment having a ternary structure as depicted by any of Figures 4A, 4B, 4C, 4D, 5A or 5B and having insulinomimetic activity.

4. A purified natural or synthetic peptide whose amino acid sequence is Sequence VIII: 
Arg-Gly-Trp-Lys-Leu-Phe-Tyr-Asn-Tyr-Ala-Leu-Val-Ile
77 78 79 80 81 82 83 84 85 86 87 88 89

Phe-Glu-Met-Thr-Asn-Leu-Lys-Asp
90 91 92 93 94 95 96 97
 or an active fragment thereof, which binds the insulin-like growth factor I molecule and which possesses IGF-I like activity.

5. An insulinomimetic drug comprising a peptide or fragment as claimed in any one of claims 1 to 4.

6. An insulinomimetic drug comprising a synthetic or purified amino acid residue sequence corresponding to any of Sequences I through IX, wherein:
Sequences I, II and III are as defined in claim 1;
Sequence IV is as follows: 
Lys-Arg-Gly-Ser-Arg-Leu-Phe-Phe-Asn-Tyr-Ala
83 84 85 86 87 88 89 90 91 92

Leu-Val-Ile-Phe-Glu-Met-Val-His-Leu-Lys-Lys
93 94 95 96 97 98 98 100 101 102
Sequences V, VI and VII are modifications of Sequence IV achieved by replacing Phe-Phe at 88-89 with Leu-Phe (Sequence V), Phe-Leu (Sequence VI) and Leu-Leu (Sequence VII);
Sequence VIII is as defined in claim 4, and
Sequence IX is as follows:

Ala-Leu-Val-Ile-Phe-Glu-Met-Val-His-Leu-Lys-Glu.

## Patentansprüche

1. Gereinigtes natürliches oder synthetisches Peptid, dessen Aminosäuresequenz die Sequenz I: 
Arg-Leu-Phe-Phe-Asn-Tyr-Ala-Leu-Val-Ile-Phe-Glu-Met-
 86  87  88  89  90  91  92  93  94  95  96  97  98

Val-His-Leu-Lys-Glu
 99 100 101 102 103
 oder die Sequenz II: 
Arg-Gly-Ser-Arg-Leu-Phe-Phe-Asn-Tyr-Ala-Leu-Val-Ile-
 83  84  85  86  87  88  89  90  91  92  93  94  95

Phe-Glu-Met-Val-His-Leu-Lys-Glu
 96  97  98  99 100 101 102 103
 oder die Sequenz III: 
(Lys)x-Arg-Leu-Phe-Phe-Asn-Tyr-Ala-Leu-Val-Ile-Phe-
86 87 88 89 90 91 92 93 94 95 96

Glu-Met-Val-His-Leu-Lys-Glu-(Lys)y
97 98 99 100 101 102 103
 ist, worin x und y jeweils 0, 1 oder 2 sind, unter der Voraussetzung, daß zumindest x oder y 1 ist,
das das menschliche Insulinmolekül bindet und insulinmimetische Aktivität hat, oder ein aktives Fragment irgendeiner der Sequenzen I, II oder III.

2. Nicht natürlich vorkommendes Peptid, dessen Aminosäuresequenz 
Arg-Gly-Ser-Arg-Leu-Phe-Phe-Asn-Tyr-Ala-Leu-Val-Ile-
 83  84  85  86  87  88  89  90  91  92  93  94  95

Phe-Glu-Met-Val-His-Leu-Lys-Glu
 96  97  98  99 100 101 102 103
 oder 
(Lys)x-Arg-Leu-Phe-Phe-Asn-Tyr-Ala-Leu-Val-Ile-Phe-
86 87 88 89 90 91 92 93 94 95 96

Glu-Met-Val-His-Leu-Lys-Glu-(Lys)y
97 98 99 100 101 102 103
 ist, worin x und y jeweils 0, 1 oder 2 ist, unter der Voraussetzung, daß zumindest ein x oder y 1 ist, oder 
Lys-Arg-Gly-Ser-Arg-Leu-Phe-Phe-Asn-Tyr-Ala-Leu-Val-
     83  84  85  86  87  88  89  90  91  92  93  94

Ile-Phe-Glu-Met-Val-His-Leu-Lys-Lys
 95  96  97  98  99 100 101 102
 oder 
Lys-Arg-Gly-Ser-Arg-Leu-Leu-Phe-Asn-Tyr-Ala-Leu-Val-
83 84 85 86 87 88 89 90 91 92 93 94

Ile-Phe-Glu-Met-Val-His-Leu-Lys-Lys
95 96 97 98 99 100 101 102
 oder 
Lys-Arg-Gly-Ser-Arg-Leu-Phe-Leu-Asn-Tyr-Ala-Leu-Val-
83 84 85 86 87 88 89 90 91 92 93 94

Ile-Phe-Glu-Met-Val-His-Leu-Lys-Lys
95 96 97 98 99 100 101 102
 oder 
Lys-Arg-Gly-Ser-Arg-Leu-Leu-Leu-Asn-Tyr-Ala-Leu-Val-
83 84 85 86 87 88 89 90 91 92 93 94

Ile-Phe-Glu-Met-Val-His-Leu-Lys-Lys
95 96 97 98 99 100 101 102
 ist.

3. Gereinigtes oder synthetisches Fragment der menschlichen Insulinrezeptor-α-Untereinheit, das Sequenz I gemäß Anspruch 1 umfaßt, wobei dieses Fragment eine ternäre Struktur, wie durch irgendeine der Figuren 4A, 4B, 4C, 4D, 5A oder 5B gezeigt, und insulinmimetische Aktivität hat.

4. Gereinigtes natürliches oder synthetisches Peptid, dessen Aminosäuresequenz die Sequenz VIII: 
Arg-Gly-Trp-Lys-Leu-Phe-Tyr-Asn-Tyr-Ala-Leu-Val-Ile-
 77  78  79  80  81  82  83  84  85  86  87  88  89

Phe-Glu-Met-Thr-Asn-Leu-Lys-Asp
 90  91  92  93  94  95  96  97
 ist, oder ein aktives Fragment davon, das das insulinähnliche Wachstumsfaktor-I-Molekül bindet und das IGF-I-ähnliche Aktivität besitzt.

5. Insulinmimetisches Arzneimittel enthaltend ein Peptid oder Fragment nach einem der Ansprüche 1 bis 4.

6. Insulinmimetisches Arzneimittel enthaltend eine synthetische oder gereinigte Aminosäurerestsequenz entsprechend irgendeiner der Sequenzen I bis IX, worin:
die Sequenzen I, II und III wie in Anspruch 1 definiert sind;
die Sequenz IV wie folgt ist: 
Lys-Arg-Gly-Ser-Arg-Leu-Phe-Phe-Asn-Tyr-Ala-
83 84 85 86 87 88 89 90 91 92

Leu-Val-Ile-Phe-Glu-Met-Val-His-Leu-Lys-Lys
93 94 95 96 97 98 99 100 101 102
die Sequenzen V, VI und VII Modifikationen der Sequenz IV sind, wobei Phe-Phe bei 88-89 durch Leu-Phe (Sequenz V), durch Phe-Leu (Sequenz VI) und durch Leu-Leu (Sequenz VII) ersetzt wurden;
die Sequenz VIII wie in Anspruch 4 definiert ist; und
die Sequenz IX wie folgt ist:

Ala-Leu-Val-Ile-Phe-Glu-Met-Val-His-Leu-Lys-Glu

## Revendications

1. Peptide naturel ou synthétique purifié, dont la séquence d'acides aminés est la séquence I : 
Arg-Leu-Phe-Phe-Asn-Tyr-Ala-Leu-Val-Ile-Phe-Glu-Met-
 86  87  88  89  90  91  92  93  94  95  96  97  98
Val-His-Leu-Lys-Glu
 99 100 101 102 103
 ou la séquence II : 
Arg-Gly-Ser-Arg-Leu-Phe-Phe-Asn-Tyr-Ala-Leu-Val-Ile-
83 84 85 86 87 88 89 90 91 92 93 94 95
Phe-Glu-Met-Val-His-Leu-Lys-Glu
96 97 98 99 100 101 102 103
 ou la séquence III : 
(Lys)x-Arg-Leu-Phe-Phe-Asn-Tyr-Ala-Leu-Val-Ile-Phe-Glu-
86 87 88 89 90 91 92 93 94 95 96 97
Met-Val-His-Leu-Lys-Glu-(Lys)y
98 99 100 101 102 103
 où x et y valent chacun 0, 1 ou 2, du moment qu'au moins x ou y vaut 1,
qui fixe la molécule de l'insuline humaine et présente une activité insulinomimétique, ou un fragment actif de l'une quelconque des séquences I, II ou III.

2. Peptide non naturel, dont la séquence de résidus d'acides aminés est : 
ARG-GLY-SER-ARG-LEU-PHE-PHE-ASN-TYR-ALA-LEU-VAL-ILE-
 83  84  85  86  87  88  89  90  91  92  93  94  95
PHE-GLU-MET-VAL-HIS-LEU-LYS-GLU
 96  97  98  99 100 101 102 103
 ou 
(LYS)X-ARG-LEU-PHE-PHE-ASN-TYR-ALA-LEU-VAL-ILE-PHE
86 87 88 89 90 91 92 93 94 95 96
GLU-MET-VAL-HIS-LEU-LYS-GLU-(LYS)Y
97 98 99 100 101 102 103
 où chacun des indices x et y vaut 0, 1 ou 2, du moment qu'au moins l'un des indices x et y vaut 1. ou 
LYS-ARG-GLY-SER-ARG-LEU-PHE-PHE-ASN-TYR-ALA-LEU-VAL-
83 84 85 86 87 88 89 90 91 92 93 94
ILE-PHE-GLU-MET-VAL-HIS-LEU-LYS-LYS
95 96 97 98 99 100 101 102
 ou 
LYS-ARG-GLY-SER-ARG-LEU-LEU-PHE-ASN-TYR-ALA-LEU-VAL-
83 84 85 86 87 88 89 90 91 92 93 94
ILE-PHE-GLU-MET-VAL-HIS-LEU-LYS-LYS
95 96 97 98 99 100 101 102
 ou 
LYS-ARG-GLY-SER-ARG-LEU-PHE-LEU-ASN-TYR-ALA-LEU-VAL-
83 84 85 86 87 88 89 90 91 92 93 94
ILE-PHE-GLU-MET-VAL-HIS-LEU-LYS-LYS
95 96 97 98 99 100 101 102
 ou

LYS-ARG-GLY-SER-ARG-LEU-LEU-LEU-ASN-TYR-ALA-LEU-VAL-
83 84 85 86 87 88 89 90 91 92 93 94
ILE-PHE-GLU-MET-VAL-HIS-LEU-LYS-LYS
95 96 97 98 99 100 101 102

3. Fragment purifié ou synthétique de la sous-unité du récepteur α de l'insuline humaine, qui comprend la séquence I selon la revendication 1, ledit fragment ayant une structure ternaire, telle que ressortant de l'une quelconque des figures 4A, 4B, 4C, 4D, 5A ou 5B et ayant une activité insulinomimétique.

4. Peptide naturel ou synthétique purifié, dont la séquence d'acides aminés est la séquence VIII : 
Arg-Gly-Trp-Lys-Leu-Phe-Tyr-Asn-Tyr-Ala-Leu-Val-Ile
77 78 79 80 81 82 83 84 85 86 87 88 89
Phe-Glu-Met-Thr-Asn-Leu-Lys-Asp
90 91 92 93 94 95 96 97
 ou un fragment actif de ce peptide, qui fixe la molécule du facteur de croissance insulinoïde I et qui possède une activité de type IGF-I.

5. Médicament insulinomimétique comprenant un peptide, ou un fragment selon l'une quelconque des revendications 1 à 4.

6. Médicament insulinomimétique comprenant une séquence de résidus d'acide aminé synthétique ou purifié correspondant à l'une quelconque des séquences I à IX, où
les séquences I, II et III sont telles que définies dans la revendication 1 ;
la séquence IV est la suivante : 
Lys-Arg-Gly-Ser-Arg-Leu-Phe-Phe-Asn-Tyr-Ala
83 84 85 86 87 88 89 90 91 92
Leu-Val-Ile-Phe-Glu-Met-Val-His-Leu-Lys-Lys
93 94 95 96 97 98 98 100 101 102
les séquences V, VI et VII sont des modifications de la séquence IV, que l'on obtient en remplaçant Phe-Phe en 88-89 par Leu-Phe (séquence V), par Phe-Leu (séquence VI) et par Leu-Leu (séquence VII)
la séquence VIII est telle que définie dans la revendication 4, et
la séquence IX est la suivante :
